# EUROPÄISCHE PATENTSCHRIFT

(11) **EP 1 399 255 B1**
(45) Veröffentlichungstag und Bekanntmachung des Hinweises auf die Patenterteilung: **15.10.2008**
(21) Anmeldenummer: 02748756.0
(22) Anmeldetag: 10.06.2002
(51) Int. Cl.: B01J 23/46, B01J 37/18, C07C 31/26, C07C 29/141

(54) **KATALYTISCHE HYDRIERUNG VON SACCHARIDEN an RUTHENIUM-KATALYSATOREN AUF EINEM SIO2-Träger**
CATALYTIC HYDROGENATION OF SACCHARIDES using RUTHENIUM catalysts on silica carrier
HYDROGENATION DES SACCHARIDES utilisant des CATALYSEURS AU RUTHENIUM SUR SUPPORT A BASE DE SILICE

(30) Priorität: 11.06.2001 DE 10128205
(43) Veröffentlichungstag der Anmeldung: 24.03.2004
(73) Patentinhaber: BASF SE, 67056 Ludwigshafen (DE)
(72) Erfinder: VANOPPEN, Dominic, 2950 Kapellen (BE); MAAS-BRUNNER, Melanie, 68165 Mannheim (DE); KAMMEL, Ulrich, 67346 Speyer (DE); ARNDT, Jan-Dirk, 68167 Mannheim (DE)
(74) Vertreter: Reitstötter - Kinzebach
(86) Internationale Anmeldenummer: PCT/EP2002/006346
(87) Internationale Veröffentlichungsnummer: WO 2002/100537

(56) Entgegenhaltungen:
- EP-A- 0 949 233
- EP-A- 0 992 475
- FR-A- 2 526 782
- US-A- 3 055 840
- US-A- 4 577 566
- US-A- 4 950 812
- US-A- 5 334 790
- US-A- 5 414 171
- HONG A J ET AL: "EFFECT OF SILICA SUPPORT ON RU-CU CLUSTER MORPHOLOGY AS DETERMINED BY CATALYTIC ACTIVITY" JOURNAL OF PHYSICAL CHEMISTRY, AMERICAN CHEMICAL SOCIETY, US, Bd. 91, Nr. 10, 7. Mai 1987 (1987-05-07), Seiten 2665-2671, XP000852586 ISSN: 0022-3654
- MOGGI P ET AL: "Ru/SiO2 catalysts prepared by the sol-gel method from Ru3(CO)12" APPLIED CATALYSIS A: GENERAL, ELSEVIER SCIENCE, AMSTERDAM, NL, Bd. 182, Nr. 2, 21. Juni 1999 (1999-06-21), Seiten 257-265, XP004271806 ISSN: 0926-860X
- ZOU W ET AL: "PRETREATMENT CHEMISTRY IN THE PREPARATION OF SILICA-SUPPORTED PT, RU, AND PT-RU CATALYSTS: AN IN SITU UV DIFFUSE REFLECTANCE STUDY" JOURNAL OF CATALYSIS, ACADEMIC PRESS, DULUTH, MN, US, Bd. 133, 1992, Seiten 202-219, XP001106141 ISSN: 0021-9517
- ROUCO ET AL J. CATAL. Bd. 84, 1983, Seiten 297 - 307, XP009010674

## Beschreibung

Die vorliegende Erfindung betrifft neue Ruthenium-Katalysatoren, ein Verfahren zu ihrer Herstellung und ihre Verwendung zur katalytischen Hydrierung von Mono- und Oligosacchariden bei der Herstellung von Zuckeralkoholen, ausgenommen von Sorbit.

Die großtechnische Herstellung von Zuckeralkoholen erfolgt in der Regel durch katalytische Hydrierung entsprechender Mono- und Disaccharide (siehe H. Schiweck et al. "Sugar Alcohols" in Ullmann's Encyclopedia of Industrial Chemistry, 5th ed. on CD-ROM). Zu diesem Zweck wurden als Katalysatoren bislang in erster Linie Nickel-Katalysatoren, z.B. Nickel-Trägerkatalysatoren oder Raney-Nickel, eingesetzt. Verschiedentlich wurde auch über den Einsatz von Ruthenium-haltigen Katalysatoren für diesen Zweck berichtet. In der Regel handelt es sich bei Ruthenium-Katalysatoren um sogenannte Trägerkatalysatoren, die Ruthenium auf einem oxidischen oder organischen Träger wie Kohle enthalten.

So beschreiben die US 4,380,680, US 4,487,980, US 4,413,152 und die US 4,471,144 Katalysatoren für die Hydrierung von Kohlehydraten zu den entsprechenden Zuckeralkoholen, die Ruthenium auf einem unter hydrothermalen Bedingungen stabilen Trägermaterial enthalten. Als hydrothermale Trägermaterialien werden alpha-Aluminiumoxid (US 4,380,680), Titan(IV)oxid (US 4,487,980), mit Titan(IV)halogenid behandeltes Aluminiumoxid (US 4,413,152) und theta-Aluminiumoxid (US 4,471,144) vorgeschlagen.

Aus der US 4,503,274 sind Katalysatoren für die Hydrierung von Kohlehydraten zu den entsprechenden Zuckeralkoholen bekannt, die durch Imprägnieren eines unter hydrothermalen Bedingungen stabilen Trägers mit einer wässrigen Rutheniumhalogenid-Lösung und anschließendes Hydrieren des Feststoffs bei Temperaturen im Bereich von 100 bis 300°C hergestellt werden.

Die US 3,963,788 beschreibt Ruthenium-Katalysatoren für die Hydrierung von Kohlehydraten, in denen das Ruthenium mit einem speziellen Zeolithen auf Basis eines Alumosilikats geträgert wurde. Die US 3,963,789 schlägt als Träger für Ruthenium-Katalysatoren kristalline Alumosilkat-Tone, insbesondere Montmorillonit vor.

Die FR-A 2526782 beschreibt die Verwendung eines durch Umsetzung von Natriumchlorid und Ruthenium via Na₂RuCl₆ hergestellten Rutheniumchlorids zur Herstellung von auf Siliziumdioxid geträgerten Ruthenium-Katalysatoren für die Hydrierung von Mono- und Oligosacchariden.

Die EP 992475 beschreibt ein Verfahren zur Herstellung von Alkoholen durch katalytische Hydrierung von Aldehyden oder Kethoen unter Verwendung trägergebundener Rutheniumkatalysatoren. Beispiel B5 beschreibt die Herstellung von Sorbitol durch Hydrierung von Glukose an einem Katalysator, der 2 % Ruthenium auf Titandioxid aufweist.

Die EP 949233 beschreibt ein verfahren zur Herstellung von zuckeralkoholen durch Hydrierung von zuckern, bei dem man den zucker mit Wasserstoff in Gegenwart eines Katalysators in Kontakt bringt, wobei der Katalysator mindestens ein Metall aus der VIII. Nebengruppe des Periodensystems umfasst.

Die parallele Anmeldung WO 02/100539 betrifft ein Verfahren zur Herstellung von Sorbit durch katalytische Hydrierung von einem Monosaccharid, das bei der Hydrierung Sorbit bildet, in flüssiger Phase, das dadurch gekennzeichnet ist, dass der verwendete Katalysator unter Rutheniumkatalysatoren ausgewählt ist, die erhältlich sind durch: i) ein oder mehrfaches Behandeln eines Trägermaterials auf Basis von amorphem siliziumdioxid mit einer halogenfreien wässrigen Lösung einer niedermolekularen Rutheniumverbindung und anschließendes Trockenen des behandelten Trägermaterials bei einer Temperatur unterhalb 200°C, ii) Reduktion des in i) erhaltenen Feststoffs mit Wasserstoff bei einer Temperatur im Bereich von 100 bis 350°C, wobei man Schritt ii) unmittelbar in Anschluss an Schritt i) durchführt.

Die parallele Anmeldung WO 02/100536 betrifft ein Verfahren zur Hydrierung ein oder mehrkerniger Aromaten unter Verwendung derartiger Katalysatoren.

Die parallele Anmeldung WO 02/100538 betrifft die Verwendung derartiger Katalysatoren zur Herstellung cycloaliphatischer Verbindungen, die Seitenketten mit Epoxidgruppen aufweisen, durch katalytische Hydrierung entsprechender aromatischer Verbindungen.

Die aus dem Stand der Technik bekannten Ruthenium-Katalysatoren weisen bei der Hydrierung von Kohlehydraten nur mässige Reaktivitäten auf mit der Folge, dass die erreichten Raum-Zeit-Ausbeuten an Zuckeralkoholen, bezogen auf den eingesetzten Katalysator gering sind. Angesichts der hohen Kosten für Ruthenium lässt daher die Wirtschaftlichkeit dieser Verfahren zu wünschen übrig. Zudem sind die Selektivitäten der Katalysatoren nicht ausreichend, so dass zusätzlicher Aufwand beim Isolieren der Wertprodukte erforderlich ist. Insbesondere wird häufig eine Epimerisierung der Hydroxygruppen beobachtet.

Der vorliegenden Erfindung liegt daher die Aufgabe zugrunde, ein verfahren für die Herstellung von Zuckeralkoholen durch katalytische. Hydrierung der entsprechenden Kohlehydrate bereitzustellen, wobei die katalystoren hohe Reaktivitäten aufweisen. Weiterhin sollten die Katalysatoren eine hohe Produkt-Selektivität aufweisen, nicht zuletzt im Hinblick auf eine kontinuierliche Ausgestaltung der Hydrierung.

Diese Aufgabe wurde überraschenderweise gelöst durch Ruthenium-Katalysatoren, die Ruthenium auf einem Trägermaterial auf Basis von amorphem Siliziumdioxid in einer Menge von 0,2 bis 7 Gew.-%, bezogen auf das Trägermaterial, enthalten, wobei das Trägermaterial zu wenigstens 90 Gew.-%, bezogen auf das Trägermaterial, aus Siliziumdioxid besteht und weniger als 10 Gew.-%, kristalline siliziumdioxid-Phasen aufweist, und wobei der Katalysator erhältlich ist durch:
i) ein oder mehrfaches Behandeln das Trägermaterials mit einer wässrigen Lösung einer Rutheniumverbindung, ausgewählt unter Ruthenium (III) nitrosylnitrat, Ruthenium (III) acetat, Natrium- und Kaliumruthenat(IV), wobei die wässrige Lösung, bezogen auf ihr Gesamtgewicht, weniger als 500 ppm Halogen enthält, und anschliessendes Trocknen des behandelten Trägermaterials bei einer Temperatur unterhalb 200°C, vorzugsweise ≤ 180°C und insbesondere ≤ 150 °C,
ii) Reduktion des in i) erhaltenen Feststoffs mit Wasserstoff bei einer Temperatur im Bereich von 100 bis 350°C, vorzugsweise 150 bis 350°C und insbesondere 200 bis 320°C,
wobei man und Schritt ii) unmittelbar im Anschluss an Schritt i) durchführt.

Die Erfindung betrifft somit die Verwendung derartiger Katalysatoren bei der Herstellung von Zuckeralkoholen durch katalytische Hydrierung der entsprechenden Mono- und Oliogsaccharide, ausgenommen die Verwendung zur Herstellung von sorbit. Die Verwendung zur Herstellung von Sorbit (=Sorbitol) ist Gegenstand der parallelen deutschen Patentanmeldung 10128203.6 und deren Nachanmeldung WO 02/100539.

Die erfindungsgemäß verwendeten Katalysatoren zeichnen sich durch eine erhöhte Aktivität und hohe Produktselektivität bei der Hydrierung von Mono- und Oligosacchariden aus.

Es wird vermutet, dass die hohe Reaktivität der erfindungsgemäß verwendeten Katalysatoren auf die besonderes gute Verteilung des Rutheniums auf der Oberfläche des Trägermaterials und auf die weitgehende Abwesenheit von Halogen im Trägermaterial zurückgeführt werden kann. Herstellungsbedingt liegt das Ruthenium in den erfindungsgemäßen Katalysatoren als metallisches Ruthenium vor. Elektronenmikroskopische Untersuchungen (TEM) der erfindungsgemäßen Katalysatoren haben gezeigt, dass das Ruthenium auf dem Trägermaterial in atomar-dipserser Form und/oder in Form von Ruthenium-Partikeln vorliegt, die nahezu ausschliesslich, d.h. zu mehr als 90 %, vorzugsweise zu mehr als 95 %, bezogen auf die Anzahl der sichtbaren Partikel, als isolierte Partikel mit Durchmessern unterhalb 10 nm, insbesondere unterhalb 7 nm vorliegen. Mit anderen Worten, der Katalysator enthält im Wesentlichen keine, d.h. zu weniger als 10 %, insbesondere weniger als 5 % Ruthenium-Partikel und/oder Agglomerate von Rutheniumpartikeln mit Durchmessern oberhalb 10 nm. Durch die Verwendung halogenfreier Rutheniumprekursoren und Lösungsmittel bei der Herstellung liegt der Chlorgehalt der erfindungsgemäßen Katalysatoren zudem unterhalb 0,05 Gew.-% (< 500 ppm), bezogen auf das Gesamtgewicht des Katalysators. Hier und im Folgenden sind alle ppm-Angaben als Gewichtsanteile zu verstehen, soweit nichts anderes angegeben ist.

Ein wesentlicher Aspekt der erfindungsgemäßen Katalysatoren ist die Verwendung eines Trägermaterials auf der Basis von amorphem Siliziumdioxid. Unter dem Begriff "amorph" versteht man in diesem Zusammenhang, dass der Anteil kristalliner Siliziumdioxid-Phasen weniger als 10 % des Trägermaterials ausmacht. Die zur Herstellung der erfindungsgemäßen Katalysatoren verwendeten Trägermaterialien können allerdings Überstrukturen aufweisen, die durch regelmässige Anordnung von Poren im Trägermaterial gebildet werden.

Als Trägermaterialien kommen grundsätzlich alle amorphen Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-% des Trägermaterials auch ein anderes oxidisches Material sein können, z.B. MgO, CaO, TiO₂, ZrO₂, Fe₂O₃ oder Alkalimetalloxid. Es versteht sich von selbst, dass das eingesetzte Trägermaterial ebenfalls halogenfrei ist, d. h. der Halogengehalt beträgt weniger als 500 ppm bezogen auf das Gesamtgewicht des Trägermaterials. Vorzugsweise enthält das Trägermaterial nicht mehr als 1 Gew.-% und insbesondere nicht mehr als 0,5 Gew.-% und insbesondere keine nachweisbaren Mengen (< 500 ppm) an Aluminiumoxid, gerechnet als Al₂O3. In einer bevorzugten Ausführungsform verwendet man Trägermaterialien, die weniger als 500 ppm Fe₂O₃ enthalten. Der Anteil an Alkalimetalloxid resultiert in der Regel aus der Herstellung des Trägermaterials und kann bis zu 2 Gew.-% betragen. Häufig beträgt er weniger als 1 Gew.-%. Geeignet sind auch Alkalimetalloxid-freie Träger (< 0,1 Gew.-%). Der Anteil an MgO, CaO, TiO₂ bzw. an ZrO₂ kann bis zu 10 Gew.-% des Trägermaterials ausmachen und beträgt vorzugsweise nicht mehr als 5 Gew.-%. Geeignet sind aber auch Trägermaterialien, die keine nachweisbaren Mengen dieser Metalloxide enthalten (< 0,1 Gew.-%).

Bevorzugt sind Trägermaterialien, die eine spezifische Oberfläche im Bereich von 50 bis 700 m²/g, insbesondere im Bereich von 80 bis 600 m²/g und speziell im Bereich von 100 bis 600 m²/g aufweisen (BET-Oberfläche nach DIN 66131). Unter den pulverförmigen Trägermaterialien sind insbesondere solche bevorzugt, deren spezifische (BET) Oberfläche im Bereich von 200 bis 600 m²/g liegt. Bei Trägermaterial in Form von Formkörpern liegt die spezifische Oberfläche insbesondere im Bereich von 100 bis 300 m²/g.

Geeignete amorphe Trägermaterialien auf Basis von Siliziumdioxid sind dem Fachmann geläufig und kommerziell erhältlich (siehe z.B. O.W. Flörke, "Silica" in Ullmann's Encyclopedia of Industrial Chemistry 5th ed. on CD-ROM). Sie können sowohl natürlichen Ursprungs als auch künstlich hergestellt worden sein. Beispiele für geeignete amorphe Trägermaterialien auf Basis von Siliziumdioxid sind Kieselgur, Kieselgele, pyrogene Kieselsäure und Fällungskieselsäure. In einer bevorzugten Ausführungsform der Erfindung weisen die Katalysatoren Kieselgele als Trägermaterialien auf.

Je nach Ausgestaltung der Hydrierverfahren, in denen die erfindungsgemäßen Katalysatoren eingesetzt werden, kann das Trägermaterial unterschiedliche Gestalt aufweisen. Sofern das Verfahren als Suspensionsverfahren ausgestaltet ist, wird man zur Herstellung der erfindungsgemäßen Katalysatoren üblicherweise das Trägermaterial in Form eines feinteiligen Pulvers einsetzen. Vorzugsweise weist das Pulver Teilchengrössen im Bereich von 1 bis 200 µm insbesondere 1 bis 100 µm auf. Bei Einsatz des Katalysators in Katalysatorfestbetten verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 1 mm bis 25 mm. Häufig werden Katalysatorstränge mit Strangdurchmessern von 2 bis 5 mm und Stranglängen von 2 bis 25 mm eingesetzt.

Der Gehalt an Ruthenium in den erfindungs gemäß verwendeten Katalysatoren liegt, bezogen auf das Gewicht des Trägermaterials und gerechnet als elementares Ruthenium, im Bereich von 0,2 bis 7 Gew.-% und insbesondere im Bereich von 0,4 bis 5 Gew.-%.

Zur Herstellung der erfindungsgemäß verwendeten Ruthenium-Katalysatoren wird das Trägermaterial zunächst mit einer halogenfreien wässrigen Lösung einer niedermolekularen Rutheniumverbindung, im Folgenden als (Ruthenium)prekursor bezeichnet, in einer Weise behandelt, dass die gewünschte Menge an Ruthenium vom Trägermaterial aufgenommen wird. Dieser Schritt wird im Folgenden auch als Tränken bezeichnet. Anschliessend wird der so behandelte Träger unter Einhaltung der oben angegebenen Temperaturobergrenzen getrocknet. Gegebenenfalls wird dann der so erhaltene Feststoff erneut mit der wässrigen Lösung des Rutheniumprekursors behandelt und erneut getrocknet. Dieser Vorgang wird so oft wiederholt, bis die vom Trägermaterial aufgenommene Menge an Rutheniumverbindung dem gewünschten Rutheniumgehalt im Katalysator entspricht.

Das Behandeln bzw. Tränken des Trägermaterials kann in unterschiedlicher Weise erfolgen und richtet sich in bekannter Weise nach der Gestalt des Trägermaterials. Beispielsweise kann man das Trägermaterial mit der Prekursor-Lösung besprühen oder spülen oder das Trägermaterial in der Prekursor-Lösung suspendieren. Beispielsweise kann man das Trägermaterial in der wässrigen Lösung des Rutheniumprekursors suspendieren und nach einer gewissen Zeit vom wässrigen Überstand abfiltrieren. Über die aufgenommene Flüssigkeitsmenge und die Ruthenium-Konzentration der Lösung kann dann der Rutheniumgehalt des Katalysators in einfacher Weise gesteuert werden. Das Tränken des Trägermaterials kann beispielsweise auch dadurch erfolgen, dass man den Träger mit einer definierten Menge der wässrigen Lösung des Rutheniumprekursors behandelt, die der maximalen Flüssigkeitsmenge entspricht, die das Trägermaterial aufnehmen kann. Zu diesem Zweck kann man beispielsweise das Trägermaterial mit der gewünschten Flüssigkeitsmenge besprühen. Geeignete Apparaturen hierfür sind die zum Vermengen von Flüssigkeiten mit Feststoffen üblicherweise verwendeten Apparate (siehe Vauck/Müller, Grundoperationen chemischer Verfahrenstechnik, 10. Auflage, Deutscher Verlag für Grundstoffindustrie, 1994, S. 405 ff.) beispielsweise Taumeltrockner, Tränktrommeln, Trommelmischer, Schaufelmischer und dergleichen.

Monolithische Träger werden üblicherweise mit den wässrigen Lösungen des Rutheniumprekursors gespült.

Die zum Tränken eingesetzten wässrigen Lösungen sind erfindungsgemäß halogenfrei, d.h. sie enthalten kein oder weniger als 500 ppm vorzugsweise weniger als 100 ppm Halogen bezogen auf das Gesamtgewicht der Lösung. Als Rutheniumprekursoren werden daher nur solche Rutheniumverbindungen eingesetzt, die kein chemisch gebundenes Halogen enthalten und die in dem wässrigen Lösungsmittel hinreichend löslich sind, und die ausgewählt sind unter Ruthenium(III)nitrosylnitrat (Ru(NO)(NO₃)₃), Ruthenium (III) acetat sowie Alkalimetallruthenaten (IV) wie Natrium- und Kaliumruthenat (IV).

Der Begriff "wässrig" bezeichnet hier Wasser sowie Mischungen von Wasser mit bis zu 50 Vol.-%, vorzugsweise nicht mehr als 30 Vol.-% und insbesondere nicht mehr als 10 Vol.-% eines oder mehrerer mit Wasser mischbarer organischer Lösungsmittel, z.B. Mischungen von Wasser mit C₁-C₄-Alkanolen wie Methanol, Ethanol, n- oder Isopropanol. Häufig setzt man Wasser als alleiniges Lösungsmittel ein. Das wässrige Lösungsmittel wird häufig zusätzlich wenigstens eine halogenfreie Säure, z.B. Salpetersäure, Schwefelsäure, Phosphorsäure oder Essigsäure, vorzugsweise eine halogenfreie Mineralsäure, zur Stabilisierung des Rutheniumprekursors in der Lösung enthalten. In vielen Fällen setzt man daher eine mit Wasser verdünnte, halogenfreie Mineralsäure, z. B. verdünnte bis halbkonzentrierte Salpetersäure als Lösungsmittel für den Rutheniumprekursor ein. Die Konzentration des Rutheniumprekursors in den wässrigen Lösungen richtet sich naturgemäss nach der aufzubringenden Menge an Rutheniumprekursor und der Aufnahmekapazität des Trägermaterials für die wässrige Lösung und liegt in der Regel im Bereich von 0,1 bis 20 Gew.-%.

Das Trocknen kann nach den üblichen verfahren der Feststofftrocknung unter Einhaltung der obengenannten Temperaturobergrenzen erfolgen. Die Einhaltung der erfindungsgemäßen Obergrenze der Trocknungstemperaturen ist für die Qualität, d.h. die Aktivität des Katalysators wichtig. Ein Überschreiten der oben angegebenen Trocknungstemperaturen führt zu einem deutlichen Verlust an Aktivität. Ein Kalzinieren des Trägers bei höheren Temperaturen, z.B. oberhalb 300°C oder gar 400°C, wie es im Stand der Technik vorgeschlagen wird, ist nicht nur überflüssig sondern wirkt sich auch nachteilig auf die Aktivität des Katalysators aus. Zur Erreichung hinreichender Trocknungsgeschwindigkeiten erfolgt die Trocknung in der Regel bei erhöhter Temperatur, z. B. bei wenigstens 40°C und insbesondere wenigstens 70°C und speziell ≥ 100°C.

Die Trocknung des mit dem Rutheniumprekursor getränkten Feststoffs erfolgt üblicherweise unter Normaldruck wobei zur Förderung der Trocknung auch ein verminderter Druck angewendet werden kann. Häufig wird man zur Förderung der Trocknung einen Gasstrom über bzw. durch das zu trocknende Gut leiten, z.B. Luft oder Stickstoff.

Die Trocknungsdauer hängt naturgemäss von dem gewünschten Grad der Trocknung und der Trocknungstemperatur ab und liegt in der Regel im Bereich von 2 h bis 30 h, vorzugsweise im Bereich von 4 h bis 15 h.

Vorzugsweise führt man die Trocknung des behandelten Trägermaterials soweit, dass der Gehalt an Wasser bzw. an flüchtigen Lösungsmittelbestandteilen vor der Reduktion ii) weniger als 5 Gew.-%, insbesondere nicht mehr als 2 Gew.-% und besonders bevorzugt nicht mehr als 1 Gew.-%, bezogen auf das Gesamtgewicht des Feststoffs ausmacht. Die angegebenen Gewichtsanteile beziehen sich hierbei auf den Gewichtsverlust des Feststoffs, bestimmt bei einer Temperatur von 300 °C, einem Druck von 1 bar und einer Dauer von 10 min. Auf diese Weise kann die Aktivität der erfindungsgemäß verwendeten Katalysatoren weiter gesteigert werden.

Vorzugsweise erfolgt das Trocknen unter Bewegen des mit der Prekursor-Lösung behandelten Feststoffs, beispielsweise durch Trocknen des Feststoffs in einem Drehrohrofen oder einem Drehkugelofen. Auf diese Weise kann die Aktivität der erfindungsgemäß verwendeten Katalysatoren weiter gesteigert werden.

Die Überführung des nach dem Trocknen erhaltenen Feststoffs in seine katalytisch aktive Form erfolgt erfindungsgemäß durch Hydrieren des Feststoffs bei den oben angegebenen Temperaturen in an sich bekannter Weise (Schritt ii)).

Zu diesem Zweck bringt man das Trägermaterial bei den oben angegebenen Temperaturen mit Wasserstoff oder einer Mischung aus Wasserstoff und einem Inertgas in Kontakt. Der Wasserstoffpartialdruck ist für das Ergebnis der Reduktion von untergeordneter Bedeutung und wird in der Regel im Bereich von 0,2 bar bis 1,5 bar variiert werden. Häufig erfolgt die Hydrierung des Katalysatormaterials bei Wasserstoffnormaldruck im Wasserstoffstrom. Vorzugsweise erfolgt das Hydrieren unter Bewegen des in i) erhaltenen Feststoffs, beispielsweise durch Hydrieren des Feststoffs in einem Drehrohrofen oder einem Drehkugelofen. Auf diese Weise kann die Aktivität der erfindungsgemäß verwendeten Katalysatoren weiter gesteigert werden.

Im Anschluss an die Hydrierung kann der Katalysator zur Verbesserung der Handhabbarkeit in bekannter Weise passiviert werden, z.B. indem man den Katalysator kurzfristig mit einem Sauerstoffhaltigen Gas, z.B. Luft, vorzugsweise jedoch mit einer 1 bis 10 Vol.-% Sauerstoff enthaltenden Inertgasmischung behandelt.

In besonderem Maße eignen sich die erfindungsgemäss verwendeten Katalysatoren zur Hydrierung der Carbonylfunktion von Mono- und oligosacchariden. Sie zeichnen sich gegenüber diesen Substraten zum einen durch sehr hohe Aktivitäten aus, so dass hohe Raum-Zeit-Ausbeuten, bezogen auf den eingesetzten Katalysator, insbesondere auf eingesetztes Ruthenium erreicht werden. Zudem werden die entsprechenden Zuckeralkohole in hohen Ausbeuten erhalten. Weiterhin ist die Produktselektivität hoch, d.h. das Auftreten von Nebenreaktion wie Epimerisierung, Decarbonylierung, Oligomerisierung und dergleichen, die zu Ausbeuteverlusten führen, ist geringer als bei den Ruthenium-Katalysatoren des Standes der Technik. Durch die erhöhte Produktselektivität verringert sich ausserdem der Aufwand für die Isolierung des gewünschten Hydrierungsprodukts. Weiterhin vereinfacht sich hierdurch eine kontinuierliche Durchführung der Reaktion. Ausserdem zeichnen sich die erfindungsgemäss verwendeten katalysatoren durch hohe Standzeiten, selbst unter den aggressiven Bedingungen einer Hydrierung in einem wässrigen Reaktionsmedium aus. Ein Aktivitätsverlust der erfindungsgemäßen Katalysatoren wird auch nach längerer Einsatzdauer im erfindungsgemäBen Hydrierverfahren, z.B. nach 1100 h, nicht oder nicht in nennenswertem Maße beobachtet.

Selbstverständlich können die in diesem Verfahren eingesetzten Katalysatoren bei nachlassender Aktivität nach den für Edelmetallkatalysatoren wie Rutheniumkatalysatoren üblichen, dem Fachmann bekannten Methoden regeneriert werden. Hier sind z. B. die Behandlung des Katalysators mit Sauerstoff wie in der BE 882279 beschrieben, die Behandlung mit verdünnten, halogenfreien Mineralsäuren, wie in der US 4,072,628 beschrieben, oder die Behandlung mit Wasserstoffperoxid, z. B. in Form wässriger Lösungen mit einem Gehalt von 0,1 bis 35 Gew.-%, oder die Behandlung mit anderen oxidierenden Substanzen, vorzugsweise in Form halogenfreier Lösungen zu nennen. Üblicherweise wird man den Katalysator nach der Reaktivierung und vor dem erneuten Einsatz mit einem Lösungsmittel, z. B. Wasser, spülen.

Die Erfindung betrifft daher auch ein Verfahren zur Herstellung von Zuckeralkohlen durch katalytische Hydrierung der korrespondierenden Mono- und Oligosaccharide, insbesondere der Mono- und Disaccharide, in flüssiger Phase an einem heterogenen Ruthenium-Katalysator, das dadurch gekennzeichnet ist, dass der heterogene Ruthenium-Katalysator ausgewählt ist unter einem erfindungsgemäßen Ruthenium-Katalysator, ausgenommen ein Verfahren zur Herstellung von Sorbit (=Sorbitol), das Gegenstand der parallelen deutschen Patentanmeldung 10128203.6 ist.

Geeignete Saccharide umfassen grundsätzlich alle bekannten Tetrosen, Pentosen, Hexosen und Heptosen und zwar sowohl Aldosen als auch Ketosen sowie deren Di- und Oligosaccaride, wobei Glucose, Fructose, Gulose und Saccharose ausgenommen sind, da sie bei Hydrierung Sorbit liefern. Zu den Monosacchariden, die im erfindungsgemässen Verfahren eingesetzt werden können, zählen beispielsweise: Erythrose, Threose, Ribose, Arabinose, Xylose, Lyxose, Allose, Altrose, Mannose, Gulose, Idose, Galactose, Talose, Erythrulose, Ribulose, Xylulose, Psicose und Tagatose und zwar sowohl die D-Form als auch die L-Form. Beispiele für Disacharide sind: Maltose, Isomaltose, Lactose, Cellobiose und Melobiose. Die Mono- und Oligosaccharide können als solche oder als Mischungen eingesetzt werden, wobei man vorzugsweise die Edukte in Reinform einsetzt.

Als geeignete Mono- und Oligosaccharide für das erfindungsgemässe Hydrierverfahren sind inbesondere die Monosaccharide Mannose für die Herstellung von Mannit, Galactose für die Herstellung von Dulcit (Galaktit) und Xylose für die Herstellung von Xylit, vorzugsweise die D-Form der Monosaccharide, sowie die Disaccharide Maltose für die Herstellung von Maltit, Isomaltulose (Palatinose) für die Herstellung von Isomaltit und Lactose für die Herstellung von Lactit zu nennen. Aber auch die anderen genannten Mono- und Oligosaccharide können in Gegenwart der erfindungsgemäßen Ruthenium-Katalysatoren zu den korrespondierenden Zuckeralkoholen hydriert werden. Dabei führt die Hydrierung von Aldosen zu Zuckeralkoholen, die hinsichtlich der OH-Gruppen die gleiche Konfiguration wie der eingesetzte Zucker aufweisen, und die Hydrierung von Furanosen in der Regel zu Gemischen zweier diastereomerer Zuckeralkohole, die sich nur in der Konfiguration des C-Atoms unterscheiden, welches in der Furanose die Carbonylfunktion trägt. Die Isolierung des jeweiligen reinen Zuckeralkohols aus dieser Mischung ist in der Regel ohne Probleme möglich.

Die Hydrierung erfolgt vorzugsweise durch Hydrieren einer Lösung des jeweiligen Mono- oder Oligosaccharids in einem wässrigen Lösungmittel. Der Begriff "wässrig" ist hierbei in der oben definierten Weise zu verstehen.

Zweckmäßigerweise wird Wasser als alleiniges Lösungsmittel verwendet, das gegebenenfalls geringe Mengen einer vorzugsweise halogenfreien Säure zur Einstellung des pH-Wertes enthält. Insbesondere setzt man das Mono- oder das Oligosacharid als wässrige Lösung ein, die einen pH-Wert im Bereich von 4 bis 10, und speziell im Bereich von 5 bis 7 aufweist.

Die Konzentration an Edukten in der flüssigen Phase kann grundsätzlich frei gewählt werden und liegt häufig im Bereich von 10 bis 80 Gew.-% und vorzugsweise im Bereich von 15 bis 50 Gew.-%, bezogen auf das Gesamtgewicht der Lösung.

Die eigentliche Hydrierung unter Verwendung der Katalysatoren erfolgt üblicherweise in Analogie zu den bekannten Hydrierverfahren für die Herstellung von Zuckeralkoholen, wie sie im eingangs genannten Stand der Technik beschrieben werden. Hierzu wird die flüssige, das Edukt enthaltende Phase mit dem Katalysator in Gegenwart von Wasserstoff in Kontakt gebracht. Der Katalysator kann dabei sowohl in der zu hydrierenden flüssigen Phase suspendiert werden (Suspensionsfahrweise) oder man führt die flüssige Phase über ein Katalysator-Fließbett (Fließbett-Fahrweise) oder ein Katalysator-Festbett (Festbettfahrweise). Die Hydrierung kann dabei sowohl kontinuierlich als auch diskontinuierlich ausgestaltet werden. Vorzugsweise führt man das erfindungsgemäße Verfahren in Rieselbettreaktoren nach der Festbettfahrweise durch. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung nach der Suspensionsfahrweise als auch zur Hydrierung am Katalysator-Fließbett und am Katalysator-Festbett sind aus dem Stand der Technik bekannt, z.B. aus Ullmanns Enzyklopädie der Technischen Chemie, 4. Auflage, Band 13, S. 135 ff. sowie aus P. N. Rylander, "Hydrogenation and Dehydrogenation" in Ullmann's Encyclopedia of Industrial Chemistry, 5th ed. on CD-ROM bekannt.

In der Regel führt man die Hydrierung bei erhöhtem WasserstoffDruck, z.B. bei einem Wasserstoffpartialdruck von wenigstens 10 bar, vorzugsweise wenigstens 20 bar und insbesondere wenigstens 40 bar durch. In der Regel wird der Wasserstoffpartialdruck einen Wert von 500 bar, insbesondere 350 bar nicht überschreiten. Besonders bevorzugt liegt der Wasserstoffpartialdruck im Bereich von 40 bis 200 bar. Die Reaktionstemperaturen betragen in der Regel wenigstens 40°C und werden häufig einen Wert von 250°C nicht überschreiten. Insbesondere führt man das Hydrierverfahren bei Temperaturen im Bereich von 80 bis 150°C durch.

Aufgrund der hohen Katalysatoraktivität benötigt man vergleichsweise geringe Mengen an Katalysator bezogen auf das eingesetzte Edukt. So wird man bei der diskontinuierlchen Suspensionsfahrweise in der Regel weniger als 1 mol-%, z.B. 10⁻³ mol-% bis 0,5 mol-% Ruthenium, bezogen auf 1 mol Zucker einsetzen. Bei kontinuierlicher Ausgestaltung des Hydrierverfahrens wird man üblicherweise das zu hydrierende Edukt in einer Menge von 0,02 bis 2 kg/(1 (Katalysator)*h) und vorzugsweise in einer Menge von 0,07 bis 0,7 kg/(1 (Katalysator)*h) über den Katalysator führen.

Die folgenden Beispiele dienen der nähren Erläuterung der Erfindung:

### I Herstellung der erfindungsgemäß verwendeten Katalysatoren

### 1. Vorschrift A: Pulverförmiger, halogenfreier Katalysator, nicht kalziniert.

Eine definierte Menge des jeweiligen Trägermaterials wurde mit der maximalen Menge einer Lösung von Ruthenium(III)nitrosylnitrat in Wasser getränkt, die vom jeweiligen Trägermaterial aufgenommen werden konnte. Die maximale vom jeweiligen Trägermaterial aufgenommene Menge war zuvor anhand einer authentischen Probe bestimmt worden. Die Konzentration der Lösung wurde jeweils so bemessen, dass die gewünschte Konzentration an Ruthenium im Trägermaterial resultierte.

Anschliessend wurde der so erhaltene Feststoff 13 h bei 120°C in einem Trockenschrank getrocknet. Der Restwassergehalt lag unter 1 Gew.-% (bestimmt als Gewichtsverlust einer 10 min bei 300°C und 1 bar getrockneten Probe).

Der so erhaltene Feststoff wurde in einem Reaktionsrohr 4 h bei 300°C im Wasserstoffstrom bei Normaldruck reduziert. Nach Abkühlen und Inertisieren mit Stickstoff wurde der Katalysator durch Überleiten von 5 Vol.-% Sauerstoff in Stickstoff über einen Zeitraum von 2 h passiviert.

### 2. Vorschrift B: Pulverförmiger, halogenfreier Katalysator, bewegt getrocknet, nicht kalziniert.

Die Herstellung erfolgte analog Vorschrift A, jedoch wurden Trocknung in einem Drehkugelofen durchgeführt. Der Restwassergehalt lag unter 1 Gew.-%.

### 3. Vorschrift C: Pulverförmiger, halogenfreier Katalysator, kalziniert.

Die Herstellung erfolgte analog Vorschrift B, jedoch wurde der nach dem Trocknen erhaltene Feststoff vor der Hydrierung 4 h auf 400°C im Luftstrom erhitzt.

### 4. Vorschrift D: Pulverförmiger, halogenhaltiger Katalysator, nicht kalziniert.

Die Herstellung erfolgte analog Vorschrift B, jedoch wurde anstelle von Ruthenium(III)nitrosylnitrat Ruthenium(III)chlorid eingesetzt.

### 5. Vorschrift E: Strangförmiger, halogenfreier Katalysator, nicht kalziniert.

Eine definierte Menge von zylindrischen Trägermaterial-Strängen (Durchmesser 4 mm, Länge 3 bis 10 mm) wurde mit der maximalen Menge einer Lösung von Ruthenium(III)nitrosylnitrat in Wasser getränkt, die vom jeweiligen Trägermaterial aufgenommen werden konnte. Die maximale vom jeweiligen Trägermaterial aufgenommene Menge war zuvor anhand einer authentischen Probe bestimmt worden. Die Konzentration der Lösung wurde jeweils so bemessen, dass die gewünschte Konzentration an Ruthenium im Trägermaterial resultierte.

Anschliessend wurden die so erhaltenen, getränkten Stränge 13 h bei 120°C in einer Drehkugelofen getrocknet. Der Restwassergehalt lag unter 1 Gew.-%.

Die so erhaltenen, getrockneten Stränge wurden in einem Drehkugelofen 4 h bei 300°C im Wasserstoffstrom bei Normaldruck reduziert. Nach Abkühlen und Inertisieren mit Stickstoff wurde der so erhaltene Katalysator durch Überleiten von 5 Vol.-% Sauerstoff in Stickstoff über einen Zeitraum von 2 h passiviert.

**Tabelle 1: Katalysatoren**

| Katalysator Nr. | Rutheniumgehalt | Vorschrift | Träger |
|---|---|---|---|
| | [Gew.-%] | | |
| K1 | 5 | B | SiO₂ Pulver¹⁾ |
| K2 (V) | 5 | D | SiO₂ Pulver¹⁾ |
| K3 | 5 | A | SiO₂ Pulver¹⁾ |
| K4 (V) | 5 | C | SiO₂ Pulver¹⁾ |
| K5 (V) | 5 | B | α-Al₂O₃ Pulver²⁾ |
| K6 (V) | 5 | B | θ-Al₂O₃ Pulver³⁾ |
| K7 (V) | 5 | B | TiO₂ Pulver⁴⁾ |
| K8 | 1 | E | SiO₂ Stränge⁵⁾ |

| | | | |
|---|---|---|---|
| V Vergleichskatalysator 1) Kieselgel-Pulver mit einem SiO₂-Gehalt > 99,95 Gew.-%, einer spezifischen BET-Oberfläche von 523 m²/g, einer Wasseraufnahme von 1,4 ml/g, einem Porenvolumen von 0,75 ml/g (ermittelt durch Stickstoffporometrie nach DIN 66134), einer definierten Porengröße von 60 Ä einer Teilchengrösse von 63 bis 200 µm; 2) alpha-Aluminiumoxid-Pulver mit einem Al₂O₃-Gehalt > 99,95 Gew.-%, einer spezifischen BET-Oberfläche von 7 m²/g, einer Wasseraufnahme von 0,84 ml/g, einer Teilchengrösse < 100 µm; 3) theta-Aluminiumoxid-Pulver mit einem Al₂O₃-Gehalt > 99,95 Gew.-%, einer spezifischen BET-Oberfläche von 80 m²/g, einer Wasseraufnahme von 1,05 ml/g, einem Porenvolumen von 0,67 ml/g (DIN 66134), einer Teilchengrösse < 100 µm; 4) Titandioxid-Pulver mit einem TiO₂-Gehalt > 99,9 Gew.-%, einer spezifischen BET-Oberfläche von 325 m²/g, einer Wasseraufnahme von 0,84 ml/g, einer Teilchengrösse < 63 µm; 5) Kieselgel-Stränge (d 4 mm, 11 bis 10 mm) aus Kieselgel mit einem SiO₂-Gehalt > 99,5 Gew.-% (0,3 Gew.-% Na₂O), einer spezifischen BET-Oberfläche von 169 m²/g, einer Wasseraufnahme von 0,95 ml/g, einem Porenvolumen von 0,7 ml/g (DIN 66134). | | | |

### II. Hydrierung von Xylose in Suspensionsfahrweise (Beispiele 1 und 2, Vergleichsbeispiele V1 bis V5)

Allgemeine Hydriervorschrift:
In einem 2,5 1 Autoklaven mit Rührer, Vorrichtungen zur Probenentnahme und einer Druckhaltung für Wasserstoff wurden 1200 ml einer 30 Gew.-% Lösung von Xylose in Wasser zusammen mit 3 g des jeweiligen Katalysators vorgelegt. Der Autoklav wurde mit Stickstoff inertisiert. Anschließend presste man 100 bar Wasserstoff auf und erwärmte den Autoklaven auf 90°C. Während der Reaktion wurde mit 1000 U/min gerührt. Zur Ermittlung des Umsatzes wurden während der Reaktion regelmäßig Proben entnommen und mittels HPLC der Gehalt an Xylose, Xylit und sonstigen Produkten bestimmt. Die Umsetzung wurde spätestens nach 10 h abgebrochen. In Tabelle 2 ist die Zeitdauer angegeben, die zur Erreichung eines maximalen Ausbeute erforderlich ist. Ausserdem ist die Selektivität hinsichtlich der Bildung von Xylit angegeben, die mit einer Genauigkeit von etwa 0,5 % (absolut) bestimmt werden konnte.

**Tabelle 2:**

| Beispiel | Kat. Nr. | Träger | t-max. | Umsatz | Selektivität |
|---|---|---|---|---|---|
| | | | [h] | [%] | [%] |
| 1 | K1 | SiO₂ Pulver | 3 | 99,85 | > 98,5 |
| V1 | K2 (V) | SiO₂ Pulver | 7 | 99,85 | > 98,5 |
| 2 | K3 | SiO₂ Pulver | 5 | 99,95 | > 98,5 |
| V2 | K4 (V) | SiO₂ Pulver | 10 | 99,96 | > 98,5 |
| V3 | K5 (V) | α-Al₂O₃ Pulver | 10 | 94,15 | 98,5 |
| V4 | K6 (V) | θ-Al₂O₃ Pulver | 10 | 99,53 | > 98,5 |
| V5 | K7 (V) | TiO₂ Pulver | 10 | 76,84 | 98,2 |

Die Ergebnisse zeigen, dass die erfindungsgemäßen Katalysatoren bessere Reaktivitäten bei vergleichbaren oder besseren Selektivitäten als nicht-erfindungsgemäße Katalysatoren.

### III Hydrierung von Mannose, Maltose und Lactose in Suspensionsfahrweise (Beispiele 3, 4 und 5)

Analog der unter II angegebenen allgemeinen Hydriervorschrift wurden wurden 1200 ml einer 30 Gew.-% Lösung des jeweiligen Mono- bzw. Disaccharids in Wasser zusammen mit 3 g des jeweiligen Katalysators bei 50 bar Wasserstoff und einer Temperatur von 120°C hydriert. Umsatz und Selekivität wurden wie unter II beschrieben mittels HPLC ermittelt. In Tabelle 3 ist die Zeitdauer angegeben, die zur Erreichung eines maximalen Umsatzes (> 99,8%) erforderlich ist. Ausserdem ist die Selektivität hinsichtlich der Bildung des gewünschten Zuckeralkohols angegeben.

**Tabelle 3:**

| Beispiel | Kat.Nr. | Edukt | Produkt | t-max. | Umsatz | Selektivität |
|---|---|---|---|---|---|---|
| | | | | [h] | [%] | [%] |
| 3 | K1 | Mannose | Mannit | 2 | >99,8 | 96 |
| 4 | K1 | Maltose | Maltit | 1 | >99,8 | 69 |
| 5 | K1 | Lactose | Lactit | 3 | >99,8 | 87 |

### III Hydrierung von Xylose und Lactose am Katalysatorfestbett (Beispiele 6 und 7)

Als Reaktor diente ein beheizbares Reaktionsrohr aus Edelstahl, das mit Katalysator K8 gefüllt war. Die Reaktionsanordnung wies eine Zulaufpumpe für die Edukte, eine Kreislaufpumpe, Vorrichtungen für die Probenentnahme sowie einen Abscheider mit Standregelung und Abgasregelung auf.

In dieser Reaktionsanordnung wurde eine 30 gew.-%ige Lösung des jeweiligen Mono- bzw. Disaccharids bei einer Temperatur von 100 °C und einem Wasserstoffdruck von 50 bar mit einer Geschwindigkeit von 50 ml/(g(Katalysator)*h) im Kreis gefahren und währenddessen mittels der unter II beschriebenen Analytik die Abnahme des Edukts, die Zunahme des Produkte und die Bildung von Nebenprodukten bestimmt. Bei erreichen eines Umsatzes von 99,4 % wurde die Umsetzung abgebrochen. Die zur Erreichung des maximalen Umsatzes erforderliche Kontaktzeit ist in Tabelle 4 zusammen mit der Selektivität angegeben. Kontaktzeit = Vol.(Lösung)/Vol.(Reaktionsrohr) *Reaktionszeit

**Tabelle 4:**

| Beispiel | Edukt | Produkt | Kontaktzeit. | Selektivität |
|---|---|---|---|---|
| | | | [h] | [%] |
| 6 | Xylose | Xylit | 0,81 | 97,2 |
| 7 | Lactose | Lactit | 1,0 | 94,1 |

## Patentansprüche

1. Verwendung eines Ruthenium-Katalysators bei der Herstellung von Zuckeralkoholen durch katalytische Hydrierung der entsprechenden Mono- und Oligosaccharide, ausgenommen zur Herstellung von Sorbit,
**dadurch gekennzeichnet, dass** der Ruthenium-Katalysator Ruthenium auf einem Trägermaterial auf Basis von amorphem Siliziumdioxid in einer Menge von 0,2 bis 7 Gew.-%, bezogen auf das Trägermaterial, enthält, wobei das Trägermaterial zu wenigstens 90 Gew.-%, bezogen auf das Trägermaterial, aus Siliziumdioxid besteht und weniger als 10 Gew.-%, kristalline Siliziumdioxid-Phasen aufweist, und wobei der Katalysator erhältlich ist durch:
i) ein oder mehrfaches Behandeln das Trägermaterials mit einer wässrigen Lösung einer Rutheniumverbindung, ausgewählt unter Ruthenium(III)nitrosylnitrat, Ruthenium(III)acetat, Natrium- und Kaliumruthenat(IV), wobei die wässrige Lösung, bezogen auf ihr Gesamtgewicht, weniger als 500 ppm Halogen enthält, und anschliessendes Trocknen des behandelten Trägermaterials bei einer Temperatur unterhalb 200°C,
ii) Reduktion des in i) erhaltenen Feststoffs mit Wasserstoff bei einer Temperatur im Bereich von 100 bis 350°C,
wobei man Schritt ii) unmittelbar im Anschluss an Schritt i) durchführt,

2. Verwendung nach Anspruch 1, **dadurch gekennzeichnet, dass** das Trägermaterial auf Basis von amorphem Siliziumdioxid eine BET-Oberfläche im Bereich von 50 bis 700 m²/g aufweist.

3. Verwendung nach einem der vorhergehenden Ansprüche, **dadurch gekennzeichnet, dass** der Ruthenium-Katalysator Ruthenium in einer Menge von 0,4 bis 5 Gew.-%, bezogen auf das Gewicht des Trägers, enthält.

4. Verwendung nach einem der vorhergehenden Ansprüche, **dadurch gekennzeichnet, dass** das Trägermaterial weniger als 1 Gew.-% Aluminiumoxid, gerechnet als Al₂O₃, enthält.

5. Verwendung nach einem der vorhergehenden Ansprüche, **dadurch gekennzeichnet, dass** der Ruthenium-Katalysator weniger als 0,05 Gew.-% Halogen, bezogen auf das Gesamtgewicht des Katalysators, enthält und aus dem Trägermaterial und elementarem Ruthenium, das auf dem Trägermaterial in atomar-disperser Form und/oder in Form von Ruthenium-Partikeln vorliegt, besteht, wobei der Katalysator im Wesentlichen keine Ruthenium-Partikel und/oder Agglomerate mit Durchmessern oberhalb 10 nm aufweist.

6. Verfahren zur Herstellung von Zuckeralkoholen durch katalytische Hydrierung der entsprechenden Mono- und Oligosaccharide in flüssiger Phase an einem heterogenen Ruthenium-Katalysator, ausgenommen ein verfahren zur Herstellung von sorbit, **dadurch gekennzeichnet, dass** der heterogene Ruthenium-Katalysator ausgewählt ist unter Ruthenium-Katalysatoren gemäß einem der Ansprüche 1 bis 5.

7. Verfahren nach Anspruch 6, **dadurch gekennzeichnet, dass** man das Mono- oder das Oligosaccharid als wässrige Lösung einsetzt, die einen pH-Wert im Bereich von 4 bis 10 aufweist.

8. Verfahren nach einem der Ansprüche 6 oder 7, **dadurch gekennzeichnet, dass** man die Hydrierung bei einem Wasserstoffpartialdruck im Bereich von 10 bis 500 bar durchführt.

9. Verfahren nach einem der Ansprüche 6 oder 7, **dadurch gekennzeichnet, dass** man die Hydrierung bei einer Temperatur im Bereich von 40 bis 250°C durchführt.

10. Verfahren nach einem der Ansprüche 6 bis 9, **dadurch gekennzeichnet, dass** man die Hydrierung an einem Katalysatorfestbett durchführt.

11. Verfahren nach einem der Ansprüche 6 bis 9, **dadurch gekennzeichnet, dass** man die Hydrierung in flüssiger Phase, enthaltend den Katalysator in Form einer Suspension, durchführt.

## Claims

1. The use of a ruthenium catalyst in the preparation of sugar alcohols by catalytic hydrogenation of the corresponding monosaccharides and oligosaccharides, with the exception of the preparation of sorbitol,
wherein the ruthenium catalyst comprises ruthenium on a support material based on amorphous silicon dioxide in an amount of from 0.2 to 7% by weight, based on the support material, with the support material comprising at least 90% by weight, based on the support material, of silicon dioxide and having less than 10% by weight of crystalline silicon dioxide phases, and the catalyst being obtainable by:
i) treating the support material with an aqueous solution of a ruthenium compound selected from among ruthenium(III) nitrosyl nitrate, ruthenium(III) acetate, sodium ruthenate (IV) and potassium ruthenate(IV), with the aqueous solution, based on its total weight, comprising less than 500 ppm of halogen, and subsequently drying the treated support material at below 200°C,
ii) reducing the solid obtained in i) by means of hydrogen at from 100 to 350°C, where step ii) is carried out directly after step i) .

2. The use as claimed in claim 1, wherein the support material based on amorphous silicon dioxide has a BET surface area in the range from 50 to 700 m²/g.

3. The use as claimed in any of the preceding claims, wherein the ruthenium catalyst comprises ruthenium in an amount of from 0.4 to 5% by weight, based on the weight of the support.

4. The use as claimed in any of the preceding claims, wherein the support material comprises less than 1% by weight of aluminum oxide, calculated as Al₂O₃.

5. The use as claimed in any of the preceding claims, wherein the ruthenium catalyst comprises less than 0.05% by weight of halogen, based on the total weight of the catalyst, and comprises the support material and elemental ruthenium which is present in atomically disperse form and/or in the form of ruthenium particles on the support material, where the catalyst comprises essentially no ruthenium particles and/or agglomerates having diameters above 10 nm.

6. A process for preparing sugar alcohols by catalytic hydrogenation of the corresponding monosaccharides and oligosaccharides in the liquid phase over a heterogeneous ruthenium catalyst, with the exception of a process for preparing sorbitol, wherein the heterogeneous ruthenium catalyst is selected from among ruthenium catalysts as claimed in any of claims 1 to 5.

7. The process as claimed in claim 6, wherein the monosaccharide or oligosaccharide is used as an aqueous solution having a pH in the range from 4 to 10.

8. The process as claimed in claim 6 or 7, wherein the hydrogenation is carried out at a hydrogen partial pressure in the range from 10 to 500 bar.

9. The process as claimed in claim 6 or 7, wherein the hydrogenation is carried out at from 40 to 250°C.

10. The process as claimed in any of claims 6 to 9, wherein the hydrogenation is carried out over a fixed catalyst bed.

11. The process as claimed in any of claims 6 to 9, wherein the hydrogenation is carried out in a liquid phase in which the catalyst is present in the form of a suspension.

## Revendications

1. Utilisation d'un catalyseur à base de ruthénium pour la fabrication de polyols par hydrogénation catalytique des mono- et oligosaccharides correspondants, à l'exception de la fabrication de sorbitol,
**caractérisée en ce que** le catalyseur à base de ruthénium contient du ruthénium sur un matériau support à base de dioxyde de silicium amorphe en une quantité de 0,2 à 7 % en poids par rapport au matériau support, ledit matériau support étant constitué d'au moins 90 % en poids, par rapport au matériau support, de dioxyde de silicium et comprenant moins de 10 % en poids de phases cristallines de dioxyde de silicium, et ledit catalyseur étant obtenu par :
i) un ou plusieurs traitements du matériau support avec une solution aqueuse d'un composé de ruthénium, choisi parmi le nitrate de nitrosyle de ruthénium (III), l'acétate de ruthénium (III), le ruthénate (IV) de sodium et de potassium, ladite solution aqueuse contenant, par rapport à son poids total, moins de 500 ppm d'halogène, puis séchage ultérieur du matériau support traité à une température inférieure à 200°C,
ii) réduction du solide obtenu en i) avec de l'hydrogène à une température de 100 à 350°C,
l'étape ii) étant réalisée immédiatement après l'étape i).

2. Utilisation selon la revendication 1, **caractérisée en ce que** le matériau support à base de dioxyde de silicium amorphe présente une surface BET de 50 à 700 m²/g.

3. Utilisation selon l'une quelconque des revendications précédentes, **caractérisée en ce que** le catalyseur à base de ruthénium contient du ruthénium en une quantité de 0,4 à 5 % en poids par rapport au poids du support.

4. Utilisation selon l'une quelconque des revendications précédentes, **caractérisée en ce que** le matériau support contient moins de 1 % en poids d'oxyde d'aluminium, calculé en tant que Al₂O₃.

5. Utilisation selon l'une quelconque des revendications précédentes, **caractérisée en ce que** le catalyseur à base de ruthénium contient moins de 0,05 % en poids d'halogène par rapport au poids total du catalyseur et est constitué du matériau support et de ruthénium élémentaire, qui est présent sur le matériau support sous forme atomique dispersée et/ou sous forme de particules de ruthénium, ledit catalyseur ne comportant essentiellement pas de particules et/ou d'agglomérats de ruthénium ayant un diamètre supérieur à 10 nm.

6. Procédé de fabrication de polyols par hydrogénation catalytique des mono- et oligosaccharides correspondants en phase liquide sur un catalyseur hétérogène à base de ruthénium, à l'exception d'un procédé de fabrication de sorbitol, **caractérisé en ce que** le catalyseur hétérogène à base de ruthénium est choisi parmi les catalyseurs à base de ruthénium selon l'une quelconque des revendications 1 à 5.

7. Procédé selon la revendication 6, **caractérisé en ce que** le mono- ou l'oligosaccharide est utilisé sous forme d'une solution aqueuse dont le pH est de 4 à 10.

8. Procédé selon l'une quelconque des revendications 6 ou 7, **caractérisé en ce que** l'hydrogénation est réalisée à une pression partielle en hydrogène de 10 à 500 bars.

9. Procédé selon l'une quelconque des revendications 6 ou 7, **caractérisé en ce que** l'hydrogénation est réalisée à une température de 40 à 250°C.

10. Procédé selon l'une quelconque des revendications 6 à 9, **caractérisé en ce que** l'hydrogénation est réalisée sur un lit catalytique solide.

11. Procédé selon l'une quelconque des revendications 6 à 9, **caractérisé en ce que** l'hydrogénation est réalisée en phase liquide contenant le catalyseur sous forme d'une suspension.
